# EUROPEAN PATENT APPLICATION

(11) **EP 3 795 154 A1**
(43) Date of publication of application: **24.03.2021**
(21) Application number: 20200021.2
(22) Date of filing: 10.11.2011
(51) Int. Cl.: A61K 31/4174, A61P 27/06

(54) **PHARMACEUTICAL COMPOSITION COMPRISING (S)-(3-(1-(1H-IMIDAZOL-4-YL)ETHYL)-2-METHYLPHENYL)METHANOL FOR USE IN TREATING OCULAR CONDITIONS**

(30) Priority: 16.11.2010 US 41418010 P
(62) Divisional of application: 16176846.0
(71) Applicant: Allergan, Inc., Irvine, CA 92612 (US)
(72) Inventor: Dibas, Mohammed I., Corona, CA, 92883 (US); Donello, John E., Dana Point, CA, 92629 (US); Gil, Daniel W., Corona Del Mar, CA, 92625 (US)
(74) Representative: Hoffmann Eitle

(57) **Abstract**

The present invention provides a pharmaceutical composition comprising (S)-(3-(1-(1H-imidazol-4-yl)ethyl)-2-methylphenyl)methanol or a salt thereof for use in a method of treating elevated intraocular pressure, glaucoma or optic neuropathy in a mammal, wherein the composition is in the form of eye drops, a slow-releasing pellet, a suspension, a gel, or an intraocular implant comprising a polymer associated with the (S)-(3-(1-(1H-imidazol-4-yl)ethyl)-2-methylphenyl)methanol or salt thereof.

## Description

### BACKGROUND OF THE INVENTION

### 1. Field of the Invention

The present invention relates to a pharmaceutical composition containing (S)-(3-(1-(1H-imidazol-4-yl)ethyl)-2-methylphenyl)methanol or a pharmaceutically acceptable salt thereof for use in treating ocular conditions.

### 2. Summary of the Related Art

[3-(1-(1H-imidazol-4-yl)ethyl)-2-methylphenyl]methanol is known as a selective modulator of the alpha 2 adrenergic receptors. Three alpha-1 and three alpha-2 adrenergic receptors have been characterized by molecular and pharmacological methods. Activation of these alpha receptors evokes physiological responses with useful therapeutic actions.

4-[1-(2,3-dimethylphenyl)ethyl]-3*H*-imidazole, generically known as, medetomidine is an alpha 2 adrenergic agonist, for use in the sedation of animals. The hydrochloride salt of the (S) enantiomer of medetomidine, generically known as dexmedetomidine, (*S*)-4-[1-(2,3-dimethylphenyl)ethyl]-3*H*-imidazole, is also indicated for use as a sedative or analgesic in cats and dogs.

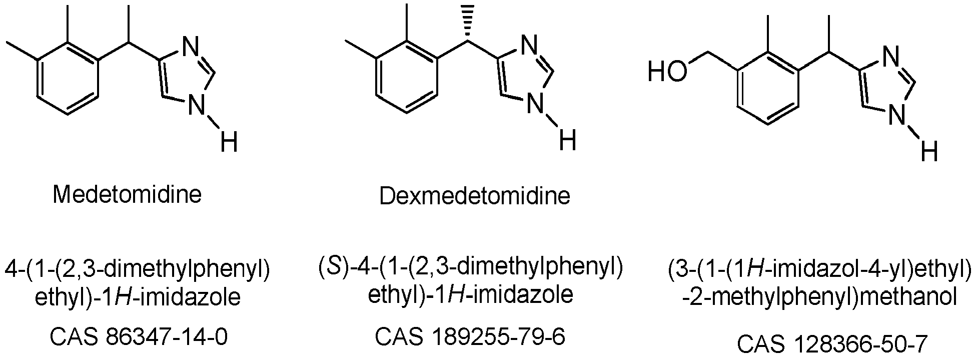

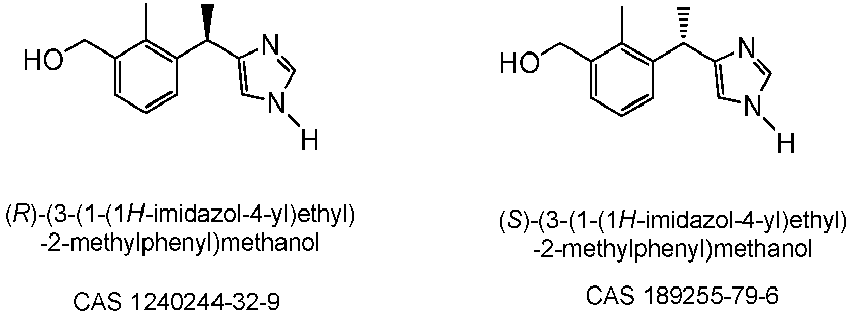

The metabolite of dexmedetomidine is (*S*)-[3-(1-(1*H*-imidazol-4-yl)ethyl)-2-methylphenyl]methanol. Journal of Chromatography, (1997), 762, 281-291 by Hui, Y.-H et al. describes (*S*)-[3-(1-(1*H*-imidazol-4-yl)ethyl)-2-methylphenyl]methanol together with its racemic mixture.

[3-(1-(1*H*-imidazol-4-yl)ethyl)-2-methylphenyl]methanol is described in "Synthesis of detomidine and medetomidine metabolites: 1,2,3-trisubstituted arenes with 4'(5')-imidazolylmethyl groups" in Journal of Heterocyclic Chemistry (1993), 30(6), (1645-1651) by Stoilov et al.

Kavanagh, et al. describe [3-(1-(1*H*-imidazol-4-yl)ethyl)-2-methylphenyl]methanol in "Synthesis of Possible Metabolites of Medetomidine {1-(2,3-dimethylphenyl)-1-[imidazol-4(5)-yl]ethane" in Journal of Chemical Research, Synopses (1993), (4), 152-3.

[3-(1-(1*H*-imidazol-4-yl)ethyl)-2-methylphenyl)methanol] is described by Salonen, et al. in "Biotransformation of Medetomidine in the Rat" in Xenobiotica (1990), 20(5), 471-80.

WO 2010093930 A1 discloses [3-(1-(1*H*-imidazol-4-yl)ethyl)-2-methylphenyl]methanol and its (*S*) and (*R*) enantiomers

### BRIEF DESCRIPTION OF THE DRAWING

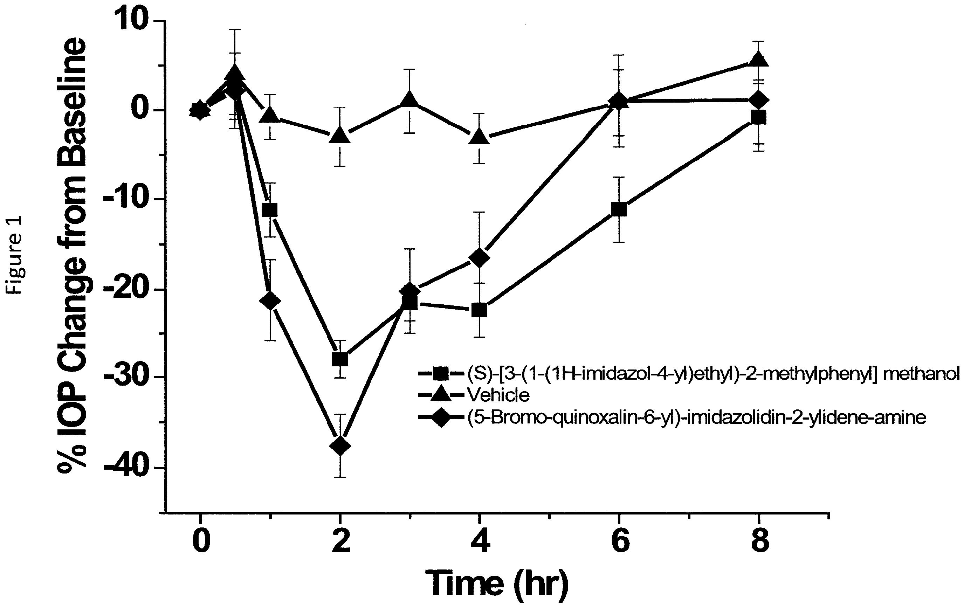

**Figure 1** shows compound (*S*)-[3-(1-(1H-imidazol-4-yl)ethyl)-2-methylphenyl] methanol (**Compound 1**) has comparable efficacy to brimonidine (Alphagan P®) and has longer intraocular pressure duration than brimonidine.

### SUMMARY OF THE INVENTION

The adrenergic Alpha-2 agonists play a key role in modulating aqueous humor formation and facilitating aqueous outflow; as a result these compounds lower intraocular pressure (IOP) in glaucomatous patients. Two drugs are currently prescribed for glaucoma patients, Apraclonidine (Iopidine®) and Brimonidine (Alphagan P® available from Allergan, Inc.). While these drugs are effective at lowering elevated intraocular pressure, Alphagan P® is the only alpha-2 adrenergic approved only for a 3 times per day dosing regime while Iopidine® is only approved for short term IOP control. Considering the aged glaucoma patient population, a 3 times per day dosing frequency is far from optimal and may result in poor patient compliance.

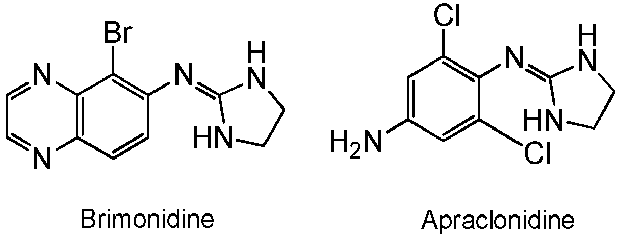

The present invention relates to a method of lowering intraocular pressure in a subject in need of such treatment, which comprises administering a pharmaceutical composition comprising a therapeutically effective amount of [3-(1-(1*H*-imidazol-4-yl)ethyl)-2-methylphenyl] methanol or its enantiomers or its tautomers or its pharmaceutical acceptable salts thereof.

In a further aspect, the present invention relates to a method of lowering intraocular pressure in a subject in need of such treatment, which comprises administering a pharmaceutical composition comprising a therapeutically effective amount of (*S*) [3-(1-(1*H*-imidazol-4-yl)ethyl)-2-methylphenyl]methanol or its tautomers or its pharmaceutical acceptable salts thereof.

In a further aspect, the present invention relates to a method of lowering intraocular pressure which comprises administering topically a therapeutically effective amount of a pharmaceutical composition comprising (*S*) (3-(1-(1*H-*imidazol-4-yl)ethyl)-2-methylphenyl)methanol or a salt thereof, to the affected eye of a patient.

In a further aspect, the present invention relates to a method of lowering intraocular pressure in a subject in need of such treatment, which comprises administering a pharmaceutical composition comprising a therapeutically effective amount of (*R*) [3-(1-(1*H*-imidazol-4-yl)ethyl)-2-methylphenyl]methanol or its tautomers or its pharmaceutical acceptable salts thereof.

In a further aspect, the present invention provides pharmaceutical compositions, containing (3-(1-(1*H*-imidazol-4-yl)ethyl)-2-methylphenyl)methanol as active ingredient for modulating the alpha 2 adrenergic receptors or pharmaceutical compositions thereof.

In a further aspect, the present invention provides pharmaceutical compositions, containing (*S*) (3-(1-(1H-imidazol-4-yl)ethyl)-2-methylphenyl)methanol as active ingredient for modulating the alpha 2 adrenergic receptors.

In a further aspect, the present invention provides pharmaceutical compositions, containing (*R*) (3-(1-(1H-imidazol-4-yl)ethyl)-2-methylphenyl)methanol as active ingredient for modulating the alpha 2 adrenergic receptors.

The pharmaceutical compositions of [3-(1-(1*H*-imidazol-4-yl)ethyl)-2-methylphenyl]methanol and its (*S*) and (*R*) enantiomers are useful for the treatment of mammals, including humans, with a range of conditions and diseases that are alleviated by alpha 2A, 2B, 2C activation, including but not limited to treating glaucoma, elevated intraocular pressure, ischemic neuropathies, optic neuropathy, pain, visceral pain, corneal pain, headache pain, migraine, cancer pain, back pain, irritable bowel syndrome pain, muscle pain and pain associated with diabetic neuropathy, the treatment of diabetic retinopathy, other retinal degenerative conditions, stroke, cognitive deficits, neuropsychiatric conditions, drug dependence and addiction, withdrawal symptoms, obsessive-compulsive disorders, obesity, insulin resistance, stress-related conditions, diarrhea, diuresis, nasal congestion, spasticity, attention deficit disorder, psychoses, anxiety, depression, autoimmune disease, Crohn's disease, gastritis, Alzheimer's, Parkinson's ALS, and other neurodegenerative diseases, dermatological conditions, skin erythema (redness) and inflammation, rosacea, acne.

The present invention relates to pharmaceutical compositions containing as active ingredient [3-(1-(1 H-imidazol-4-yl)ethyl)-2-methylphenyl] methanol or containing as active ingredient (*S*) [3-(1-(1 H-imidazol-4-yl)ethyl)-2-methylphenyl] methanol or containing as active ingredient (*R*) [3-(1-(1 H-imidazol-4-yl)ethyl)-2-methylphenyl] methanol for treatment of diseases and or alleviations of conditions which are responsive to treatment by agonists of alpha adrenergic receptors.

These pharmaceutical compositions may be used to lower IOP in glaucoma and other ophthalmologic diseases.

In a further aspect of the invention, there is provided a method of lowering intraocular pressure of a patient in need thereof which comprises, consists essentially of or consists of administering a therapeutically effective amount of a pharmaceutical composition comprising, consisting essentially of or consisting of a therapeutically effective amount of (*S*)-[3-(1-(1H-imidazol-4-yl)ethyl)-2-methylphenyl] methanol or a salt thereof to the affected eye of said patient, as a single dose, wherein the affected eye maintains an intraocular pressure less than the baseline intraocular pressure for at least eight (8) hours and preferably at least ten (10) hours and more preferably at least twelve (12) hours, from the time of administration.

In a further aspect of the invention, there is provided a method of lowering intraocular pressure of a patient in need thereof which comprises of administering a therapeutically effective amount of a pharmaceutical composition comprising a therapeutically effective amount of (*S*)-[3-(1-(1 H-imidazol-4-yl)ethyl)-2-methylphenyl] methanol or a salt thereof to the affected eye of said patient, as a single dose, wherein the affected eye maintains an intraocular pressure less than the baseline intraocular pressure for at least eight (8) hours.

In a further aspect of the invention, there is provided a method of lowering intraocular pressure of a patient in need thereof which comprises of administering a therapeutically effective amount of a pharmaceutical composition comprising a therapeutically effective amount of (*S*)-[3-(1-(1H-imidazol-4-yl)ethyl)-2-methylphenyl] methanol or a salt thereof to the affected eye of said patient, as a single dose, wherein the affected eye maintains an intraocular pressure less than the baseline intraocular pressure for at least ten (10) hours.

In a further aspect of the invention, there is provided a method of lowering intraocular pressure of a patient in need thereof which comprises of administering a therapeutically effective amount of a pharmaceutical composition comprising a therapeutically effective amount of (*S*)-[3-(1-(1H-imidazol-4-yl)ethyl)-2-methylphenyl] methanol or a salt thereof to the affected eye of said patient, as a single dose, wherein the affected eye maintains an intraocular pressure less than the baseline intraocular pressure for at least twelve (12)hours.

The term "baseline", as used herein, refers to the intraocular pressure measurement taken for the untreated eye.

The term "subject", as used herein, refers to a human patient.

In a further aspect of the invention, there is provided a method of lowering intraocular pressure in a patient in need thereof which comprises, consists essentially of or consists of administering a therapeutically effective amount of a pharmaceutical composition comprising, consisting essentially of or consisting of a therapeutically effective amount of [3-(1-(1*H*-imidazol-4-yl)ethyl)-2-methylphenyl] methanol, or the enantiomers thereof, or the tautomers thereof, or pharmaceutically acceptable salts thereof.

In a further aspect of the invention, there is provided a method of lowering intraocular pressure in a patient in need thereof which comprises, consists essentially of or consists of administering a therapeutically effective amount of a pharmaceutical composition comprising, consisting essentially of or consisting of a therapeutically effective amount of (*S*) [3-(1-(1*H*-imidazol-4-yl)ethyl)-2-methylphenyl] methanol, or the tautomers thereof, or pharmaceutically acceptable salts thereof.

In a further aspect of the invention, there is provided a method of lowering intraocular pressure in a patient in need thereof which comprises, consists essentially of or consists of administering a therapeutically effective amount of a pharmaceutical composition comprising, consisting essentially of or consisting of a therapeutically effective amount of (*R*) [3-(1-(1*H*-imidazol-4-yl)ethyl)-2-methylphenyl] methanol, or the tautomers thereof, or pharmaceutically acceptable salts thereof.

In a further aspect of the invention there is provided a method of treating a patient having elevated intraocular pressure with an effective amount of (*S*)-[3-(1-(1H-imidazol-4-yl)ethyl)-2-methylphenyl] methanol to lower intraocular pressure, wherein the improvement comprises, consists essentially of or consists of lowering the elevated intraocular pressure for a prolonged period of at least eight (8) hours and preferably at least ten (10) hours and more preferably at least twelve (12) hours, by administering to the affected eye of said patient a single dose of a composition comprising, consisting essentially of or consisting of a therapeutically effective amount of (*S*)-[3-(1-(1H-imidazol-4-yl)ethyl)-2-methylphenyl] methanol.

In a further aspect of the invention there is provided a method of treating a patient having elevated intraocular pressure with an effective amount of (*S*)-[3-(1-(1H-imidazol-4-yl)ethyl)-2-methylphenyl] methanol to lower intraocular pressure, wherein the improvement consists of lowering the elevated intraocular pressure for a prolonged period of at least eight (8) hours.

In a further aspect of the invention there is provided a method of treating a patient having elevated intraocular pressure with an effective amount of (*S*)-[3-(1-(1H-imidazol-4-yl)ethyl)-2-methylphenyl] methanol to lower intraocular pressure, wherein the improvement consists of lowering the elevated intraocular pressure for a prolonged period of at least ten (10) hours.

In a further aspect of the invention there is provided a method of treating a patient having elevated intraocular pressure with an effective amount of (*S*)-[3-(1-(1H-imidazol-4-yl)ethyl)-2-methylphenyl] methanol to lower intraocular pressure, wherein the improvement consists of lowering the elevated intraocular pressure for a prolonged period of at least twelve (12) hours.

In a further aspect of the invention, there is provided a method of lowering intraocular pressure of a patient in need thereof which comprises administering a therapeutically effective amount of a composition comprising (*S*)-[3-(1-(1H-imidazol-4-yl)ethyl)-2-methylphenyl] methanol, to the affected eye of said patient, once or twice daily, preferably once daily, wherein the affected eye maintains an intraocular pressure less than the baseline intraocular pressure, throughout the day.

In a further method of the invention, said intraocular pressure is lowered for at least eight (8) hours subsequent to administration.

In a further method of the invention, said intraocular pressure is lowered for at least ten (10) hours subsequent to administration.

In a further method of the invention, said intraocular pressure is lowered for at least twelve (12) hours subsequent to administration.

In a further method according to the present invention, the composition that is used, as a single dose, to lower intraocular pressure for at least eight (8) hours and preferably at least ten (10) hours and more preferably for at least twelve (12) hours, may comprise from about 0.01 to about 5 percent by weight, preferably from about 0.01 to about 2 percent by weight, more preferably from about 0.05 to about 1 percent by weight, (*S*)-[3-(1-(1H-imidazol-4-yl)ethyl)-2-methylphenyl] methanol in a pharmaceutically-acceptable vehicle. Said composition is preferably formulated as an eye drop suitable for topical administration.

In forming compositions for topical administration, the pharmaceutical compositions are preferably formulated as a solution in water at a pH of about 5.5 to about 8.0, e.g. about 6.9. While the precise regime is left to the discretion of the clinician, it is recommended that the solution be topically applied by placing one drop in each eye one or two times, preferably once a day. Other ingredients which may be desirable to use in the ophthalmic preparations used in the method of the present invention include preservatives, co-solvents and viscosity building agents; bodium chloride, potassium chloride, calcium chloride dihydrate, magnesium chloride hexahydrate, boric acid and sodium borate decahydrate (as buffering agents) and purified water (Clinical Ocular Pharmacology By Jimmy D. Bartlett, Siret D. Jaanus, 2008, p 266).

Preservatives are thus required to prevent microbial contamination during use. Suitable preservatives include: stabilized oxychloro complex (sold under the trademark Purite™), stabilized chlorine dioxide,,benzalkonium chloride, thimerosal, chlorobutanol, methyl paraben, propyl paraben, phenylethyl alcohol, edetate disodium, sorbic acid, Onamer M, or other agents known to those skilled in the art (Review of Ophthalmology, June 2001, Robert Noecker, MD). A common side-effect of these preservatives is burning.

A further method of the present invention offers the improvement of exposing the patient to less preservative, since the (*S*)-[3-(1-(1H-imidazol-4-yl)ethyl)-2-methylphenyl] methanol containing compositions are administered only once or at most, twice a day, unlike the prior art alpha-2 adrenergic agonists which require three doses, daily, to control elevated intraocular pressure. Typically, for the compositions utilized in the method of the present invention, the effective concentration of the preservative will range from about 0.001% to about 1 % by weight, preferably from about 0.01% to about 0.5%, by weight. In particular stabilized oxychloro complex (Purite ®) will range from about 0.001 to about 0.01 %, by weight.

The solubility of the components of the present compositions may be enhanced by a surfactant or other appropriate co-solvent in the composition. Such cosolvents include polysorbate 20, 60, and 80, Pluronic ® F-68, F-84 and P-103, cyclodextrin, Solutol, or other agents known to those skilled in the art. Typically such co-solvents are employed at a level of from about 0.01% to about 2% by weight.

Increase of the viscosity of the aqueous solutions may be desirable to increase ocular absorption of the active compound, to decrease variability in dispensing the formulation, to decrease physical separation of components of a suspension or emulsion of the formulation and/or to otherwise improve the ophthalmic formulation. Such viscosity building agents include as examples polyvinyl alcohol, polyvinyl pyrrolidone, methyl cellulose, hydroxypropyl methylcellulose, hydroxyethyl cellulose, carboxymethyl cellulose, hydroxypropyl cellulose or other agents known to those skilled in the art. Such agents are typically employed at a level of from about 0.01% to about 2% by weight.

The following formulations are representative ophthalmic compositions of the invention for topical use when indicated for treating elevated intraocular pressure associated with glaucoma. In one example, the free base of (*S*)-[3-(1-(1H-imidazol-4-yl)ethyl)-2-methylphenyl] methanol was dissolved in sterile distilled water, hydrochloric acid was added and the hydrochloric salt of the compound was formed *in situ.* The solution was titrated with sodium hydroxide until the pH of the solution reached about 8.0. The final concentration of (*S*)-[3-(1-(1H-imidazol-4-yl)ethyl)-2-methylphenyl] methanol is about 1% by weight. In another example, the free base of (*S*)-[3-(1-(1 H-imidazol-4-yl)ethyl)-2-methylphenyl] methanol was dissolved in sterile distilled water with boric acid, benzalkonium chloride and glycerin.

Compounds of the invention are formulated as pharmaceutical compositions. "Pharmaceutical composition," as used here, means a composition that is suitable for administering to human patients for the treatment of disease. In a further embodiment, therefore, the compounds of the invention are formulated as pharmaceutically acceptable salts and further include one or more pharmaceutically acceptable excipients.

"Pharmaceutically acceptable salt" refers to those salts which retain the biological effectiveness and properties of the free base and which are obtained by reaction with inorganic acids such as hydrochloric acid, hydrobromic acid, sulfuric acid, nitric acid, phosphoric acid, methanesulfonic acid, ethanesulfonic acid, p-toluenesulfonic acid, salicylic acid and the like. The acid addition salt form of (S)-[3-(1-(1 H-imidazol-4-yl)ethyl)-2-methylphenyl] methanol that occurs in its free form as a base, can be obtained by treating the free base with an appropriate acid such as an inorganic acid, for example hydrochloric acid, hydrobromic acid, sulfuric acid, phosphoric acid, nitric acid and the like; or an organic acid such as for example, acetic, hydroxyacetic, propanoic, lactic, pyruvic, malonic, fumaric acid, maleic acid, oxalic acid, tartaric acid, succinic acid, malic acid, ascorbic acid, benzoic acid, tannic acid, pamoic acid, citric, methylsulfonic, ethanesulfonic, benzenesulfonic, formic and the like (Handbook of Pharmaceutical Salts, P.Heinrich Stahal& Camille G. Wermuth (Eds), Verlag Helvetica Chemica Acta-Zürich, 2002, 329-345).

The compounds can also be administered as pharmaceutically acceptable quaternary salts known by those skilled in the art, which specifically include, but not limiting to the quaternary ammonium salt of the formula -NY⁺Z⁻, wherein Y is hydrogen, alkyl, or benzyl, and Z is a counterion, including but not limited to, chloride, bromide, iodide, -O-alkyl, toluenesulfonate, methylsulfonate, sulfonate, phosphate, or carboxylate (such as fumarate, benzoate, succinate, acetate, glycolate, maleate, malate, fumarate, citrate, tartrate, ascorbate, benzoate, cinnamoate, mandeloate, benzyloate, and diphenylacetate).

In a further embodiment of the invention, there are provided pharmaceutical compositions including at least one compound of the invention in a pharmaceutically acceptable carrier thereof. The phrase "pharmaceutically acceptable" means the carrier, diluent or excipient must be compatible with the other ingredients of the formulation and not deleterious to the recipient thereof.

Pharmaceutical compositions of the present invention can be used in the form of a solid, a solution, an emulsion, a dispersion, a patch, a micelle, a liposome, and the like, wherein the resulting composition contains one or more compounds of the present invention, as an active ingredient, in admixture with an organic or inorganic carrier or excipient suitable for enteral or parenteral applications. Invention compounds may be combined, for example, with the usual non-toxic, pharmaceutically acceptable carriers for tablets, pellets, capsules, suppositories, solutions, emulsions, suspensions, and any other form suitable for use. The carriers which can be used include but are not limited to, glucose, lactose, gum acacia, gelatin, mannitol, starch paste, magnesium trisilicate, talc, corn starch, keratin, colloidal silica, potato starch, urea, medium chain length triglycerides, dextrans, and other carriers suitable for use in manufacturing preparations, in solid, semisolid, or liquid form. In addition auxiliary, stabilizing, thickening and coloring agents and perfumes may be used. Invention compounds are included in the pharmaceutical composition in an amount sufficient to produce the desired effect upon the process or disease condition.

Pharmaceutical compositions containing invention compounds may be in a form suitable for oral use, for example, as tablets, troches, lozenges, aqueous or oily suspensions, dispersible powders or granules, emulsions, hard or soft capsules, or syrups or elixirs. Compositions intended for oral use may be prepared according to any method known in the art for the manufacture of pharmaceutical compositions and such compositions may contain one or more agents selected from the group consisting of a sweetening agent such as sucrose, lactose, or saccharin, flavoring agents such as peppermint, oil of wintergreen or cherry, coloring agents and preserving agents in order to provide pharmaceutically elegant and palatable preparations. Tablets containing invention compounds in admixture with non-toxic pharmaceutically acceptable excipients may also be manufactured by known methods. The excipients used may be, for example, (1) inert diluents such as calcium carbonate, lactose, calcium phosphate or sodium phosphate; (2) granulating and disintegrating agents such as corn starch, potato starch or alginic acid; (3) binding agents such as gum tragacanth, corn starch, gelatin or acacia, and (4) lubricating agents such as magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monostearate or glyceryl distearate may be employed. In some cases, formulations for oral use may be in the form of hard gelatin capsules wherein the invention compounds are mixed with an inert solid diluent, for example, calcium carbonate, calcium phosphate or kaolin. They may also be in the form of soft gelatin capsules wherein the invention compounds are mixed with water or an oil medium, for example, peanut oil, liquid paraffin or olive oil.

The pharmaceutical compositions may be in the form of a sterile injectable suspension. This suspension may be formulated according to known methods using suitable dispersing or wetting agents and suspending agents. The sterile injectable preparation may also be a sterile injectable solution or suspension in a non-toxic parenterally-acceptable diluent or solvent, for example, as a solution in 1,3-butanediol. Sterile, fixed oils are conventionally employed as a solvent or suspending medium. For this purpose any bland fixed oil may be employed including synthetic mono- or diglycerides, fatty acids (including oleic acid), naturally occurring vegetable oils like sesame oil, coconut oil, peanut oil, cottonseed oil, etc., or synthetic fatty vehicles like ethyl oleate or the like. Buffers, preservatives, antioxidants, and the like can be incorporated as required.

The present invention concerns also the use of 3-(1-(1H-imidazol-4-yl)ethyl)-2-methylphenyl] methanol or its enantiomers or its tautomers or a pharmaceutically acceptable salt thereof, for the manufacture of a medicament for the therapeutic application. The present invention concerns also the method for manufacturing a medicament intended for therapeutic application wherein the compound is 3-(1-(1H-imidazol-4-yl)ethyl)-2-methylphenyl]methanol or its enantiomers or its tautomers or a pharmaceutically active derivative or salt thereof is used.

The present invention concerns also the use of a (*S*)-[3-(1-(1H-imidazol-4-yl)ethyl)-2-methylphenyl] methanol or a pharmaceutically acceptable salt thereof, for the manufacture of a medicament for the therapeutic application. The present invention concerns also the a method for manufacturing a medicament intended for therapeutic application wherein the compound is (*S*)-[3-(1-(1H-imidazol-4-yl)ethyl)-2-methylphenyl] methanol or a pharmaceutically active derivative or salt thereof is used.

The present invention concerns also the use of a (*R*)-[3-(1-(1H-imidazol-4-yl)ethyl)-2-methylphenyl] methanol or a pharmaceutically acceptable salt thereof, for the manufacture of a medicament for the therapeutic application. The present invention concerns also the a method for manufacturing a medicament intended for therapeutic application wherein the compound is (*R*)-[3-(1-(1H-imidazol-4-yl)ethyl)-2-methylphenyl] methanol or a pharmaceutically active derivative or salt thereof is used.

Since individual subjects may present a wide variation in severity of symptoms and each drug has its unique therapeutic characteristics, the precise mode of administration and dosage employed for each subject is left to the discretion of the practitioner. The patient will be administered the compound orally in any acceptable form, such as a tablet, liquid, capsule, powder and the like, or other routes may be desirable or necessary, particularly if the patient suffers from nausea. Such other routes may include, without exception, transdermal, parenteral, subcutaneous, intranasal, via an implant stent, intrathecal, intravitreal, topical to the eye, back of the eye, intramuscular, intravenous, and intrarectal modes of delivery. The actual amount of the compound to be administered in any given case will be determined by a physician taking into account the relevant circumstances, such as the severity of the condition, the age and weight of the patient, the patient's general physical condition, the cause of the condition, and the route of administration. Additionally, the formulations may be designed to delay release of the active compound over a given period of time, or to carefully control the amount of drug released at a given time during the course of therapy.

(*S*)-[3-(1-(1H-imidazol-4-yl)ethyl)-2-methylphenyl] methanol and its pharmaceutically-acceptable salts have extended alpha-2 adrenergic receptor agonist activity in lowering intraocular pressure and may be administered through different routes, including but not limited to topical eye drops, direct injection, application at the back of the eye or formulations that may further enhance the long duration of actions such as a slow releasing pellet, suspension, gel, or sustained delivery devices such as any suitable drug delivery system (DDS) known in the art.

While topical administration is preferred, this compound may also be used in an intraocular implant as described in U.S. Published Patent Application 20050244463. Such biocompatible intraocular implants include (*S*)-[3-(1-(1H-imidazol-4-yl)ethyl)-2-methylphenyl] methanol and a polymer associated with (S)-[3-(1-(1 H-imidazol-4-yl)ethyl)-2-methylphenyl] methanol to facilitate release thereof into an eye for an extended period of time.

The present invention is not to be limited in scope by the exemplified embodiments, which are only intended as illustrations of specific aspects of the invention. Various modifications of the invention, in addition to those disclosed herein, will be apparent to those skilled in the art by a careful reading of the specification, including the claims, as originally filed. It is intended that all such modifications will fall within the scope of the appended claims.

The following assays and animal models are used to demonstrate the potency and selectivity of the compounds according to the invention.

### Example 1

### In-Vivo IOP Compound Screening

The experimental animals used, were Normotensive male Dutch-Belted rabbits (Myrtle's Rabbitry) over 6 months in age (n= 4/compound/dose screened). A single drop (50 µl) of the drug formulation, was administered topically by pipette onto the right eye (treated eye) at approximately 0700 hours. IOP of the rabbits (treated and untreated eyes) was measured 0 hours before and at 0.5, 1, 2, 3, 4, 6 and 8 hours after topical eyedrop administration. IOP at the time of eyedrop administration (0 hours) was used as a baseline value. Prior to the tonometric measurements, 0.05% proparacaine (50 µl) was administered to each eye. Tonometric IOP measurements were obtained with a Mentor Pneumontonmeter. Additionally, all studies were masked. At least 1 week of wash-out time was allowed for each rabbit between dosings. All animals were examined for sedation, ocular irritation, and changes in pupil diameter throughout the course of the experiments.

(*S*)-[3-(1-(1H-imidazol-4-yl)ethyl)-2-methylphenyl] methanol (Compound 1) at 0.15% concentration, lowered IOP for at least 6 hours in the treated eye of DB Rabbits, whereas the effect of brimonidine (0.15%) was minimal at 6 hrs. (S)-[3-(1-(1H-imidazol-4-yl)ethyl)-2-methylphenyl] methanol showed very little effect in the contralateral eye whereas brimonidine showed a strong contralateral effect. The data is shown in **Figure 1****.**

### Example 2

### FLIPR Ca⁺² Influx Assay

HEK 293 cells stably expressing the bovine α_{1A} receptor, human alpha 2A receptor and the chimeric G protein G_{qi5}, are plated in poly-D-lysine coated 384-well plates at 20,000 - 40,000 cells per well and grown overnight in DMEM supplemented with 10% fetal bovine serum. For FLIPR (fluorometric image plate reader) evaluation, cells are washed twice with HBSS/HEPES Buffer (1X Hanks Buffered Salt Solution, 20 mM HEPES, pH 7.4) prior to the addition of Fluo-4-AM (4 uM Fluo-4-AM, 0.04% pluronic acid in HBSS/HEPES Buffer), a calcium-sensitive dye. Cells are loaded with dye for 40 minutes at 37°C, then washed 4 times with HBSS/HEPES Buffer. For both the agonist and antagonist assay, the test compounds are tested between 0.64 nM - 10,000 nM.

For an agonist assay, the reaction is initiated by the addition of the appropriate dilutions of compounds and the transient calcium signal captured. The peak height of the calcium curve is determined and utilized for calculation of EC₅₀ and efficacy using ActivityBase. Norepinephrine is the standard full agonist used for evaluating alpha-1 and alpha-2 receptor activity.

**Table 1**

| *In Vitro* Pharmacology of (*S*)-[3-(1-(1H-imidazol-4-yl)ethyl)-2-methylphenyl] methanol at adrenergic receptor subtypes. | | | |
|---|---|---|---|
| **Entry** | **Compound** | **FLIPR Assay** | |
| | | **a1A** | **a2A** |
| 1 | Brimonidine | 600-2400 (0.3) | 5 (0.95) |
| 2 | (*S*)-[3-(1-(1H-imidazol-4-yl)ethyl)-2-methylphenyl] methanol (Compound 1) | 340-2400 (0.7) | 25 (0.9) |

| | | | |
|---|---|---|---|
| EC50 in nM (efficacy) | | | |

## Claims

1. A pharmaceutical composition comprising (S)-(3-(1-(1H-imidazol-4-yl)ethyl)-2-methylphenyl)methanol or a salt thereof for use in a method of treating elevated intraocular pressure, glaucoma or optic neuropathy in a mammal, wherein the composition is in the form of eye drops, a slow-releasing pellet, a suspension, a gel, or an intraocular implant comprising a polymer associated with the (S)-(3-(1-(1H-imidazol-4-yl)ethyl)-2-methylphenyl)methanol or salt thereof.

2. A pharmaceutical composition for use according to Claim 1, wherein the composition further comprises from about 0.001% to about 1% by weight of a preservative.

3. A pharmaceutical composition for use according to Claim 2, wherein the composition comprises from about 0.01% to about 0.5% by weight of a preservative.

4. A pharmaceutical composition for use according to any preceding claim, wherein the composition further comprises from about 0.01% to about 2% by weight of a co-solvent.

5. A pharmaceutical composition for use according to any preceding claim, wherein the composition further comprises from about 0.01% to about 2% by weight of a viscosity-building agent.
